Europäisches Patentamt

European Patent Office

Office européen des brevets

(11) Publication number: **0 275 719**
**A2**

## (12) EUROPEAN PATENT APPLICATION

(21) Application number: 87311526.5

(22) Date of filing: 30.12.87

(51) Int. Cl.⁴: **A61K 7/42**

(30) Priority: 30.12.86 US 947728

(43) Date of publication of application:
27.07.88 Bulletin 88/30

(84) Designated Contracting States:
AT BE CH DE FR GB GR IT LI LU NL SE

(71) Applicant: **CHARLES OF THE RITZ GROUP LTD.**
**40 West 57th Street**
**New York, NY 10019(US)**

(72) Inventor: **Georgalas, Arthur C. W.**
**12 Bellevue Avenue**
**Leonardo New Jersey 07737(US)**

(74) Representative: **Beresford, Keith Denis Lewis et al**
**BERESFORD & Co. 2-5 Warwick Court High Holborn**
**London WC1R 5DJ(GB)**

(54) Ultraviolet absorbing compounds and formulations including same.

(57) Improved substantive ultraviolet absorbing compound, and a sunscreen or sunblock formulation including such a compound, characterized by an ultraviolet absorbing agent having an acid group, in combination with a fatty amine. The acid-containing ultraviolet absorbing agents are selected from the groups consisting of UV-A absorbing agents including sulisobenzone and thioctic acid, and UV-B absorbing agents including 2-phenylbenzimidazole-5-sulfonic acid, salicylic acid, methoxycinnamic acid, cinnamic acid and ferrulic acid. The fatty amine is selected from the group consisting of lauramidopropyl dimethylamine, oleamidopropyl dimethylamine, ricinoleamidopropyl dimethylamine, stearamidoethyl ethanolamine, stearamidoethyl diethylamine, cocoyl imidazoline, and alkyldimethylamine. The resulting improved ultraviolet absorbing compound and formulations into which it is incorporated have enchanced substantivity and thereby greater water resistance.

EP 0 275 719 A2

# ULTRAVIOLET ABSORBING COMPOUNDS AND FORMULATIONS INCLUDING SAME

The present invention relates to improved ultraviolet absorbing compounds and more particularly concerns sunscreen or sunblock formulations which include such compounds and which have enhanced substantivity.

Sunscreen and sunblock formulations provide excellent protection against severe sun burning of exposed skin. These formulations include agents which can absorb ultraviolet light at various wavelengths. For example, ultraviolet radiation from the sun reaching the earth's atmosphere is typically divided into two ranges; the UV-A range (wavelengths of 320 to 400 nanometers) and the UV-B (wavelength of 290 to 320 nanometers). Numerous UV-A and UV-B absorbing compounds or agents are known in the art.

Since different degrees of energy are absorbed by skin exposed to these varying wavelengths of ultraviolet radiation, sunscreen preparations can be formulated to extend the period of time it takes the sun to produce a sunburn by varying the amounts of the UV-A and UV-B absorbers incorporated therein.

However, many commercially available sunscreen or sunblock formulations suffer from the disadvantage that they are washed off the skin during bathing in pool water or ocean water. This results in certain areas of the skin being exposed to greater amounts of ultraviolet radiation than intended.

Improved ultraviolet absorbing compounds resistant to water wash-off, i.e. having enhanced substantivity, would be a useful addition to the art.

In accordance with the present invention an improved ultraviolet absorbing compound, and a sunscreen or sunblock formulation including such a compound, are provided. The improved ultraviolet absorbing compound comprises, in combination, an ultraviolet absorbing agent having an acid group and a fatty amine. The resulting improved ultraviolet absorbing compound and formulations into which it is incorporated have enhanced substantivity and thereby greater water resistance.

Further in accordance with the present invention there is provided a sunscreen or sunblock formulation comprising one or more ultravilet absorbing agents, which agents include an acid group, in combination with a fatty amine, water and optionally one or more emollients, emulsifiers, preservatives, antioxidants, fragrances, coloring agents or chelating agents.

It has been found that sunscreen or sunblock formulations are provided with greater substantivity when they are formulated to include ultraviolet absorbing compounds having an acid group and one or more fatty amines. In fact, the reaction products of the acid-containing UV absorbers and fatty amines also possess this enhanced substantivity. Therefore, these reaction products represent improved ultraviolet absorbing compounds and, as such, are considered part of the present invention.

In one embodiment, a sunscreen or sunblock formulation according to the present invention includes compounds which have an acid group and which absorb light in the UV-A (320-400 nm) and UV-B (290 to 320 nm) regions, one or more fatty amines and typically also contains water, emollients, emulsifiers, thickeners, preservatives, coloring agents, fragrances, antioxidants and the like.

The formulation of the present invention is preferably an oil-in-water type emulsion since this type of emulsion affords a better cosmetic feel to the product. However, the product may also be formulated as a water-in-oil emulsion, cream base or oil base. Depending upon the choice of ingredients, the composition has a semi-solid, cream-like consistency which can be packaged in a plastic squeeze tube or it has a lotion type consistency which can be packaged in a plastic squeeze container.

As mentioned above, the present invention relies on the combination of a UV absorbing agent having an acid group, with a fatty amine. The fatty amine may be a primary, secondary or tertiary amine derived from, for example, tallow, coconut oil, soybean oil, marine oils, rapeseed and the like. It can also be a primary ether amine, i.e. a fatty amine derived from an alcohol instead of from a fatty acid. The fatty amine can also be a lipophilic derivative of a fatty amidopropyl dimethylamine base.

Suitable fatty amines include lauramidopropyl dimethylamine (Lexamine L-13), oleamidopropyl dimethylamine (Lexamine O-13), ricinoleamidopropyl dimethylamine (Lexamine R-13), stearamidoethyl ethanolamine (Chemical base 39), stearamidoethyl diethylamine (Chemical base 6532), cocoyl imidazoline (Miramine CC), and alkyldimethylamine (ADMATM2).

In addition to the fatty amines, other amines or bases may be employed to enhance the solubility of the UV-absorbers in the present formulations. These include, but are not limited to, water-soluble amines (e.g. triethanolamine), ammonia or alkali hydroxides.

In accordance with the present invention the fatty amine is typically combined with both the UV-A and UV-B absorbers having acid groups. Alternatively, the fatty amine can be combined with one or the other of the UV absorbers. The improved UV absorbing compound of the present invention may comprise the fatty amine in a molar ratio to the UV absorbers (A and/or B) between about 0.25:1 and 2:1 and preferably from about 0.5:1 to 1.2:1.

A sunscreen or sunblock formulation according to the present invention may comprise between about 0.5 and 20 percent by weight, and preferably includes between about 1 and 8 percent by weight of the fatty amines.

Suitable UV-A absorbers having acid groups include sulisobenzone (2-hydroxy-4-methoxybenzophenone-5-sulfonic acid; Uvinul MS-40), thioctic acid (lipoic acid), and the like. The sulisobenzone is the preferred UV-A absorber. The desired UV-A absorber will be present in the final product in an amount of from about 0.25 to about 20 percent by weight, and preferably in an amount of from about 1 to about 8 percent by weight of the total formulation. The amount will vary according to the particular absorber selected and whether the formulation is intended to prevent, minimize or permit tanning.

Suitable UV-B absorbers having acid groups include 2-phenylbenzimidazole-5-sulfonic acid (Eusolex® 232), p-aminobenzoic acid, salicylic acid, methoxycinnamic acid, cinnamic acid, ferrulic acid and the like. The Eusolex® 232 is the preferred UV-B absorber. The desired UV-B absorber will be present in the final product in an amount of from about 0.25 to about 20 percent by weight, and preferably from about 1 to 8 percent by weight of the total formulation. The amount will vary according to the particular absorber selected and whether the formulation is intended to prevent, minimize or permit tanning.

The total amount of UV absorbers included within the formulation will be between about 0.5 and 20 percent by weight; which amount will determine whether the formulation is a sunblock or a sunscreen.

In addition to the fatty amine and UV absorbers, the present formulation can include from about 60 to about 95 percent by weight of a solvent, e.g. ethanol, isopropanol, etc.

Alternatively, in addition to the fatty amine and UV absorbers, the formulation may contain from about 50 percent to about 90 percent and preferably from about 60 to about 80 percent by weight of water, from about 1 percent to about 20 percent and preferably from about 1 to about 5 percent by weight of emollients, from about 1 percent to about 10 percent and preferably from about 1 to about 5 percent by weight of emulsifiers, from about 0.05 to about 2 percent and preferably from about 0.1 to about 1 percent by weight of preservatives and antioxidants, and less than about 1 percent by weight of fragrance, coloring agents and chelating agents (such as EDTA).

Suitable emollients include mineral oil, avocado oil, squalane, octyl palmitate, cocoa butter, sesame oil, petrolatum, propylene glycol dicaprylate/dicaprate, isopropyl myristate, dimethicone (e.g. Silicon 225), etc. The formulation will preferably contain a mixture of several of these emollients or others which are approved for cosmetic use.

Suitable emulsifiers include polyethylene glycol 20 sorbitan monolaurate (Polysorbate 20), glyceryl stearate, polyethylene glycol 100 stearate, polyethylene glycol 20 stearyl ether (Brij 78, Steareth 20), polysorbate 80 (Tween 80), etc. The formulation will preferably contain a mixture of two or more of these emulsifiers or others which are approved for cosmetic use.

Suitable preservatives include imidazolidinyl urea (Germall 115), methylparaben (Tegosept M), quaternium-15 (N-(3-chloroallyl)hexaminium chloride, Dowicil 200), propylparaben (Tegosept P), dimethyldimethoyl hydantoin (Glydant), benzyl alcohol and/or phenoxyethanol, etc., and a preferred antioxidant is a mixture of butylated hydroxyanisole, propylene glycol, propyl gallate and citric acid (Tenox 2). The formulation will preferably contain the antioxidant mixture and one or more of the preservatives or any other preservatives and antioxidants approved for cosmetic use.

As discussed above, by varying the percentage of ingredients the formulation can be obtained in a lotion or semi-solid form. For example, in formulating the product as a lotion, water would be included at from about 60 percent to 65 percent by weight of the final product and one or more humectants such as propylene glycol, glycerin, 1,3-butylene glycol, sorbitol, polyethylene glycols (for example, Carbowax 400), could be included at up to about 20 percent by weight of the final product.

The composition of the invention will optionally include a thickener in an amount within the range of from about 0.05 to about 1 percent and preferably from about 0.05 to about 0.3 percent by weight. Examples of thickeners which may be employed herein include, but are not limited to, stearic acid, magnesium aluminum silicate, stearoxydimethicone, hydroxyethy cellulose, hydroxypropyl cellulose or xanthan gum.

Skin conditioning agents which may optionally be present in the composition of the invention include allatoin, d-or dl-panthenol, hydrolyzed animal protein and the like. Such conditioning agents may be present in an amount within the range of from about 0.01 to about 5 percent and preferably from about 0.05 to about 2 percent by weight depending upon the ultimate use of the skin preparation.

The process techniques will vary depending upon the particular ingredients employed. For example, in one such process a first aqueous blend can be prepared by mixing the UV absorbing agents, a water soluble organic base such as triethanolamine, a chelating agent such as EDTA and other water soluble

3

ingredients, if desired, into the deionized water. This is mixed thoroughly and typically heated, for example, to about 70°C. The fatty amine is added, by itself or with an alcohol, to complete the aqueous blend. A second blend is prepared by mixing an emolient, e.g. dimethicone emulsion, with a small amount of deionized water and one or more emulsifiers, if desired. The first and second blends were sweep mixed at room temperature to form an emulsion. A third blend comprising preservatives and other desired ingredients in a small amount of deionized water is thereafter added .to the emulsion at room temperature to complete the desired formulation.

A preferred sunblock formulation in accordance with the present invention will include about 70 percent by weight of water, about 4 percent by weight of Eusolex® 232, about 4.5 percent by weight of triethanolamine, about 5 percent by weight of methoxycinnamic acid, about 2.5 percent by weight of salicylic acid, about 1 percent by weight of sulisobenzone, about 4 percent by weight of cocoyl imidazoline, about 3 percent by weight of emolients, about 5 percent by weight of emulsifiers, about 0.35 percent by weight of thickener, and up to 1 percent by weight of combined preservatives, antioxidants, fragrances and chelating agents.

A preferred sunscreen formulation which protects, but still permits gradual tanning, according to the present invention includes about 80 percent by weight of water, about 4 percent by weight of Eusolex® 232, about 1 percent by weight of sulisobenzone, about 6 percent by weight of cocoyl imidazoline, about 3 percent by weight of emolients, about 5 percent by weight of emulsifiers, about 0.35 percent by weight of thickener and up to about 1 percent by weight of combined preservatives, antioxidants, fragrances, and chelating agents.

The present invention will be further described by reference to the following Examples. It should be understood, however, that the present invention is not to be limited to the details described therein.

Example 1

A sunblock formulation in the form of a lotion having the following composition was prepared as described below.

| Ingredient | Percent by Weight |
|---|---|
| **Blend I** | |
| Deionized Water | 70.0 |
| Eusolex® 232 (UV-B absorber) | 4.0 |
| Methoxycinnamic acid (UV-B absorber) | 5.0 |
| Salicylic acid (UV-B absorber) | 2.5 |
| Sulisobenzone (Uvinul MS-40; UV-A absorber) | 1.0 |
| Triethanolamine (for solubility of UV absorbers) | 4.5 |
| Cocoyl imidazoline (Miramine CC; fatty amine) | 4.0 |
| EDTA (chelating agent) | 0.10 |
| **Blend II** | |
| Dimethicone emulsion (Silicon 225; emolient) | 5.0 |
| Deionized water | 5.0 |
| **Blend III** | |
| Germall II (preservative) | 0.2 |
| Glydant (preservative) | 0.3 |
| Deonized water | 1.0 |

Aqueous blend I was prepared by mixing the UV absorbers, triethanolamine, EDTA and fatty amine into the water. The mixing was continued at about 70°C until clear.

Thereafter, the dimethicone emulsion and water of blend II were added to the so-formed blend I at room temperature and mixed thoroughly. Finally, blend III comprising the preservatives and water were added at room temperature to provide the desired formulation.

Example 2

A sunscreen formulation in the form of a lotion having the following composition was prepared as described in Example 1.

| Ingredient | Percent by Weight |
|---|---|
| **Blend I** | |
| Deionized Water | 80.0 |
| Eusolex® 232 (UV-B absorber) | 4.0 |
| Sulisobenzone (Uvinul MS-40; UV-A absorber) | 1.0 |
| Cocoyl imidazoline (Miramine CC; fatty amine) | 6.0 |
| EDTA (chelating agent) | 0.1 |
| **Blend II** | |
| Dimethicone emulsion (Silicon 225; emolient) | 5.0 |
| Deionized water | 5.0 |
| **Blend III** | |
| Germall II (preservative) | 0.2 |
| Glydant (preservative) | 0.3 |
| Deionized water | 1.0 |

Example 3

A sunscreen formulation in the form of a lotion having the following composition was prepared as described below.

| Ingredient | Percent by Weight |
|---|---|
| **Blend I** | |
| Eusolex® 232 (UV-B absorber) | 4.0 |
| Sulisobenzone (Uvinul MS-40; UV-A absorber) | 2.0 |
| Stearamidoethyl diethylamine (Chemical base 6532; fatty amine) | 8.0 |
| Ethanol | 86.0 |

The above ingredients were heated to about 70°C and mixed thoroughly. Upon cooling to room temperature the mixture formed the lotion of the present invention. Upon evaporation of the alcohol a viscous yellow paste was formed which was found to be a water-dispersible substantive UV-absorbing compound according to the present invention, which could, in turn, be used in other formulations.

## Claims

1. A substantive ultraviolet absorbing compound comprising one or more ultraviolet absorbing agents, including an acid group, in combination with a fatty amine.

2. The compound of claim 1 wherein said acid-containing ultraviolet absorbing agents are selected from the groups consisting of UV-A absorbing agents including sulisobenzone and thioctic acid, and UV-B absorbing agents including 2-phenylbenzimidazole-5-sulfonic acid, salicylic acid, methoxycinnamic acid, cinnamic acid and ferrulic acid.

3. The compound of claim 1 or 2 wherein said fatty amine is selected from the group consisting of lauramidopropyl dimethylamine, oleamidopropyl dimethylamine, ricinoleamidopropyl dimethylamine, stearamidoethyl ethanolamine, stearamidoethyl diethylamine, cocoyl imidazoline, and alkyldimethylamine.

4. The compound of claim 1, 2 or 3 wherein the molar ratio of the fatty amine to the absorbing agent including the acid group is from about 0.25:1 to about 2:1.

5. The compound of claim 4 wherein the molar ratio of the fatty amine to the absorbing agent including the acid group is from about 0.5:1 to about 1.2:1.

6. A sunscreen or sunblock formulation comprising the substantive ultraviolet absorbing compound of claim 1 in combination with a solvent.

7. The formulation of claim 6 wherein said solvent is an alcohol.

8. A sunscreen or sunblock formulation comprising one or more ultraviolet absorbing agents which include an acid group, in combination with a fatty amine, water and optionally one or more emollients, emulsifiers, preservatives, antioxidants, fragrances, coloring agents or chelating agents.

9. The formulation of claim 8 wherein said one or more ultraviolet absorbing agent are selected from the group of UV-A absorbing agents consisting of sulisobenzone and thioctic acid and one or more ultraviolet absorbing agents selected from the group of UV-B absorbing agents consisting of 2-phenylbenzimidazole-5-sulfonic acid, salicylic acid, methoxycinnamic acid, cinnamic acid and ferrulic acid.

10. The formulation of claim 8 or 9 wherein said fatty amines are selected from the group consisting of lauramidopropyl dimethylamine, oleamidopropyl dimethylamine, ricinoleamidopropyl dimethylamine, stearamidoethyl ethanolamine, stearamidoethyl diethylamine, cocoyl imidazoline, and alkyldimethylamine.

11. The formulation of claim 8, 9 or 10 comprising from about 0.25 to about 20 percent by weight of UV-A absorbing agents which include an acid group, from about 0.25 to about 20 percent by weight of UV-B absorbing agents which include an acid group, from about 0.5 to about 20 percent by weight of a fatty amine, from about 50 to about 90 percent by weight of water, from about 1 to about 20 percent by weight of an emollient, from about 1 to about 10 percent by weight of an emulsifier, from about 0.05 to about 2 percent by weight of preservatives and antioxidants, and less than about 1 percent by weight of fragrances, coloring agents and chelating agents.

12. The formulation of claim 8, 9 or 10 comprising from about 1 to about 8 percent by weight or UV-A absorbing agents which include an acid group, from about 1 to about 8 percent by weight of UV-B absorbing agents which include an acid group, from about 1 to about 8 percent by weight of a fatty amine, from about 60 to about 80 percent by weight of water, from about 1 to about 5 percent by weight of an emollient, from about 1 to about 5 percent by weight of an emulsifier, from about 0.1 to about 1 percent by weight of preservatives and antioxidants, and less than about 1 percent by weight of fragrances, coloring agents and chelating agents.

13. The formulation of claim 8 in the form of a maximum protection sunblock comprising about 70 percent by weight of water, about 4 percent by weight of 2-phenylbenzimidazole-5-sulfonic acid, about 4.5 percent by weight of triethanolamine, about 5 percent by weight of methoxycinnamic acid, about 2.S percent by weight of salicylic acid, about 1 percent by weight of sulisobenzone, about 4 percent by weight of cocoyl imidazoline, about 3 percent by weight of emolients, about 5 percent by weight of emulsifiers, about 0.35 percent by weight of thickener, and up to 1 percent by weight of combined preservatives, antioxidants, fragrances and chelating agents.

14. The formulation of claim 8 in the form of a sunscreen which permits gradual tanning comprising about 80 percent by weight of water, about 4 percent by weight of 2-phenylbenzimidazole-5-sulfonic acid, about 1 percent by weight of sulisobenzone, about 6 percent by weight of cocoyl imidazoline, about 3 percent by weight of emolients, about 5 percent by weight of emulsifiers, about 0.35 percent by weight of thickener and up to about 1 percent by weight of combined preservatives, antioxidants, fragrances, and chelating agents.

15. The formulation of claim 7 comprising about 2 percent by weight of sulisobenzone, about 4 percent by weight of 2-phenylbenzimidazole-5-sulfonic acid, about 8 percent by weight of stearamidoethyl diethylamine, and about 86 percent by weight of ethanol.